(19)

Europäisches
Patentamt
European
Patent Office
Office européen
des brevets

(11)  **EP 2 716 770 A2**

(12)  **EUROPEAN PATENT APPLICATION**

(43) Date of publication:
**09.04.2014 Bulletin 2014/15**

(51) Int Cl.:
***C12Q 1/68*** *(2006.01)*

(21) Application number: **13188069.2**

(22) Date of filing: **16.01.2009**

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **17.01.2008 FI 20085039**
**12.02.2008 US 28057 P**

(62) Document number(s) of the earlier application(s) in accordance with Art. 76 EPC:
**09702038.2 / 2 240 605**

(71) Applicant: **Mobidiag Oy**
**02150 Espoo (FI)**

(72) Inventors:
• **Mäki, Minna-Mari**
**02150 Espoo (FI)**

• **Pellosniemi, Jaakko**
**02150 Espoo (FI)**
• **Piiparinen, Pasi**
**02150 Espoo (FI)**

(74) Representative: **Kolster Oy Ab**
**Iso Roobertinkatu 23**
**PO Box 148**
**00121 Helsinki (FI)**

Remarks:
This application was filed on 10-10-2013 as a divisional application to the application mentioned under INID code 62.

(54)  **Nucleic acid probes for species-specific detection of infectious bacteria**

(57)     The invention relates to improved nucleic acid probes and to broad-range primers that are useful in the identification of bacterial species and the diagnosis of bacterial infections. Especially, the invention relates to specific nucleic acid probes that originate from hyper-variable regions situated between the conserved sequences of topoisomerase genes, gyrB and parE, of infection-causing bacteria. The invention also relates to improved broad-range primers originating from the conserved regions of topoisomerase genes. In addition, the invention relates to the use of these improved nucleic acid probes and broad-range primers in the diagnosis of bacterial infections as well as to methods in which these nucleic acid probes and broad-range primers are used.

EP 2 716 770 A2

**Description**

FIELD OF THE INVENTION

The invention relates to nucleic acid probes and to improved broad-range primers that are useful in the identification of bacterial species and in the diagnosis of bacterial infections.

BACKGROUND OF THE INVENTION

Sepsis is a severe life-threatening systemic disease resulting from an infection of the bloodstream by toxin-producing bacteria. In sepsis, dysfunction of the circulatory system may lead to multiple organ failures and to a drop in the blood pressure, resulting in shock. Worldwide, sepsis is the most common cause of death in intensive care units with a mortality rate ranging from 20% to 60%.

Diagnosis of sepsis is mainly based on bacterial culture testing. Bacterial culture testing is, however, relatively slow, and diagnostic methods based on cultivation usually give results only days, sometimes up to weeks after sampling. Furthermore, cultivation of bacteria is not always successful under laboratory conditions. This can be a consequence of either the fact that the culture method used is not applicable for the bacterial species in question, or the fact that the patient has received antimicrobial therapy before the sample is taken. Molecular methods based on amplification and hybridization of nucleic acids aim to solve the above-mentioned problems of bacterial culture testing. In such methods, the pathogen is simultaneously detected and identified, resulting in more rapid diagnostics and obviating the need for additional culture tests.

One molecular method used in bacterial diagnostics is the so-called broad-range PCR (polymerase chain reaction) based on the use of broad-range primers. The most common broad-range PCR methods use primers that recognize conserved DNA sequences of bacterial chromosomal genes encoding ribosomal RNA (16S rDNA/rRNA or 23S rDNA/rRNA). In bacterial identification based on broad-range PCR, the actual identification step is carried out by cloning and sequencing the amplified PCR product (e.g., EP-B1 613 502, US patent publications 6,001,564, and 2002/0055101).

The broad-range bacterial PCR method has been to some extent applied to clinical bacterial diagnostics, although it is best suited for the identification of bacterial and fungal species from culture isolates. For this purpose commercial tests, such as MicroSeq® (Applied Biosystems), have been developed. These tests, however, are not widely used because sequencing of PCR products is time-consuming and labor-intensive. The assays themselves and the necessary equipment, such as sequencing instruments, are expensive, and performing the tests requires specially trained personnel. Furthermore, in polymicrobial infections extensive cloning experiments may be needed.

The use of ribosomal RNA also includes some drawbacks. Distinguishing the related bacterial species from each other with the help of rRNA molecules is difficult, because the sequences of these molecules do not contain enough variable regions when they are compared with each other. Even if differences between various bacterial species may be found, these variable sites are generally divided across the whole rRNA molecule (e.g., the length of 16S rRNA is about 1500 nucleotides). This limits the use of molecular methods for diagnostics. In practice, the related bacterial species can thus be distinguished from each other only by sequencing the whole rRNA encoding gene. Even this approach is not always sufficient for bacterial identification. In phylogenetic analysis, *gyrB* gene has reported to be more discriminative in bacterial identification than for example 16S rRNA gene (Dauga, Int. J. Syst. Evol. Microbiol., 52: 531-547, 2002).

International patent publication WO2004046379 discloses tools and methods for diagnosis of bacterial infections, especially respiratory tract infections. This publication discloses broad-range primers that originate from conserved regions of genes encoding topoisomerases, especially the *gyrB* and *parE* genes, and bacterial species-specific oligonucleotide probes that originate from hyper-variable regions situated between the conserved regions. Using these broad-range primers and bacterial species-specific probes, single or even multiple infection-causing bacteria can be simultaneously detected and identified. The probes, however, cover only certain bacterial species. Furthermore, they do not allow identification of all bacterial strains due to variation occurring in their DNA sequences. Some of the probes also cross-react to some extent with untargeted clinically relevant pathogens leading to potential misidentification. In addition, the probes may possess low hybridization efficiency. In regard to the primers, they are highly degenerated demanding a complicated oligosynthesis with possible batch-to-batch variation. Also, the concentration of a single oligo variant in a PCR reaction mixture may be low and thus, the amplification of some strains could be inefficient. Thus, there is a real need in the art for improved tools and methods for bacterial diagnostics.

BRIEF DESCRIPTION OF THE INVENTION

The purpose of the present invention is to provide improved tools and methods, which are useful in bacterial diagnostics of infectious diseases, especially sepsis, but which lack the drawbacks of bacterial diagnostics described above. In particular, the purpose of the invention is to provide novel tools and methods, which are useful in detecting and identifying bacteria based on molecular methods, the tools and methods being sensitive, effective, and species or higher-level taxa specific, and being capable of identifying selectively the desired bacteria. A further purpose of the invention is to provide methods, by which it is possible to detect and identify simultaneously multiple infectious bacteria, especially those that cause sepsis, more accurately than previously possible, whereby for instance appropriate and effective antimicrobial therapy can be prescribed to the patient at an earlier stage of the infection so that the duration of the infection becomes shorter and the risk of potentially harmful, even life-threatening complications is reduced.

The present invention relates to a method of detecting and identifying infection-causing bacteria in a sample, which method comprises the steps of

a) amplifying DNA from said sample using a DNA primer pair comprising a sequence that hybridizes with the conserved regions of genes encoding topoisomerases, wherein at least one primer selected from a group consisting of SEQ ID NOs: 1 and 2 and reversed or complementary sequences thereof,

b) contacting the amplified DNA with at least one oligonucleotide probe which hybridizes with a bacterial species or higher-level taxon specific sequence of a hyper-variable region that is situated between the regions with which said DNA primers hybridize, comprising a sequence selected from a group consisting of SEQ ID NOs: 3 to 121 and reversed or complementary sequences thereof, and

c) detecting any hybridization complexes formed and identifying said infection-causing bacteria based on said detection of said hybridization complex.

In one preferred embodiment of the method according to the present invention, the sample to be analyzed is a clinical sample.

In another preferred embodiment, the infection-causing bacteria are bacteria that cause sepsis. Examples of such bacteria belong taxonomically to Bacillales, Bacteroidetes, Clostridia, Lactobacillales, and Proteobacteria (Beta, Epsilon, Gamma). More specifically, the bacteria to be detected and identified are selected from the Proteobacteria, specifically Betaproteobacteria including *Neisseria meningitidis,* Epsilonproteobacteria including *Campylobacter jejuni,* and Gammaproteobacteria including *Acinetobacter baumannii, Enterobacter aerogenes, Enterobacter cloacae, Escherichia coli, Haemophilus influenzae, Klebsiella oxytoca, Klebsiella pneumoniae, Proteus mirabilis, Proteus vulgaris, Pseudomonas aeruginosa, Salmonella enterica* subspecies *enterica, Serratia marcescens,* and *Stenotrophomonas maltophilia,* the Lactobacillales including *Enterococcus faecalis, Enterococcus faecium, Streptococcus agalactiae, Streptococcus dysgalactiae, Streptococcus pneumoniae,* and *Streptococcus pyogenes,* the Bacillales including *Listeria monocytogenes, Staphylococcus aureus, Staphylococcus epidermidis,* Bacteroidetes including *Bacteroides fragilis,* and Clostridia including *Clostridium perfringens.* In one embodiment, *Staphylococcus* genus, *Proteus* genus and/or *Enterobacteriaceae* family members are detected and identified.

In a further preferred embodiment, a first DNA primer comprising at least one sequence depicted in SEQ ID NO: 1 and a second DNA primer comprising at least one sequence depicted in SEQ ID NO: 2 are used for amplifying the DNA.

Furthermore, one preferred embodiment of the method of the invention comprises amplification the DNA from a biological specimen by PCR. In another preferred embodiment of the method of the invention, a suitably labeled nucleotide or a labeled primer is used in the amplification of DNA from a biological specimen in order to generate a detectable target strand.

In still another preferred embodiment of the method of the invention, the amplified and possibly labeled target DNA is contacted with at least one species or higher-level taxa specific oligonucleotide probe according to the present invention selected from a group consisting of SEQ ID NOs: 3 to 121 and reversed or complementary sequences thereof placed on a suitable solid support, such as treated glass surface or silicon based biochip, or in a solution. In a still further embodiment, microarray technology is used.

The present method may consist of the steps described above or it may comprise for example DNA amplification with further primers and/or contacting the amplified DNA with further probes. In some embodiments, the method may also comprise further steps, such as washing and incubation steps.

The present invention also relates to a combination of oligonucleotide probes hybridizing with hyper-variable regions situated between the conserved sequences of topoisomerase genes of infection-causing bacteria, especially sepsis, and comprising any one of the sequences identified with SEQ ID NOs: 3 to 121, and/or reverse or complementary sequences thereof, or functional fragments thereof. Said combination is useful in the detection and identification of infection-causing bacteria and comprises at least two said oligonucleotide probe sequences. In one embodiment, the combination comprises at least one probe comprising a sequence selected from a group consisting of SEQ ID NOs: 39 to 44 and reversed or complementary sequences thereof, and at least one probe comprising a sequence selected from a group consisting of SEQ ID NOs: 3 to 38, SEQ ID NOs: 45 to 121, and reversed or complementary sequences thereof.

In some embodiments, the combination of oligonucleotides probes is specific for one or more taxonomical groups or bacterial species. In one embodiment, said combination comprises all said oligonucleotides probes.

The present invention still further relates to oligonucleotide probes that hybridize with hyper-variable regions situated between the conserved sequences of topoisomerase genes of infection-causing bacteria, especially sepsis, and comprise any one of the sequences identified with SEQ ID NOs: 3 to 121, and/or reverse or complementary sequences thereof, or functional fragments thereof. The present invention also relates to the use of the above-mentioned improved oligonucleotide probe sequences in the detection, identification, or classification of bacterial species. The length of oligonucleotide probe sequence of the invention is preferably 15 to 60, more preferably 15 to 40, and most preferably 20 to 30 nucleotides.

The present invention further relates to DNA primers that comprise sequences that hybridize with conserved regions of genes encoding topoisomerases of infection-causing bacteria, selected from a group consisting of SEQ ID NOs: 1 and 2 and reversed or complementary sequences and functional fragments thereof. The present invention also relates to the use of the above-mentioned improved primers in the amplification of topoisomerase genes, especially genes encoding the gyrB and the parE proteins.

The present invention further relates to a kit for use in the detection and identification of infection-causing bacteria, especially those causing sepsis, comprising

> a) at least one DNA primer according to the present invention,
> b) at least one bacterial species or higher-level taxa specific oligonucleotide probe according to the present invention, optionally attached on a solid support,
> c) optionally, at least one positive and/or negative control probe sequence, and
> d) optionally, reagents required in the amplification, purification, hybridization, washing, and/or detection steps.

In one embodiment, the kit consists of the components described above or it may comprise further primers and/or probes, as well as other appropriate reagents.

In further embodiment, the kit comprises probes specific for one or more taxonomical groups or bacterial species.

BRIEF DESCRIPTION OF THE DRAWINGS

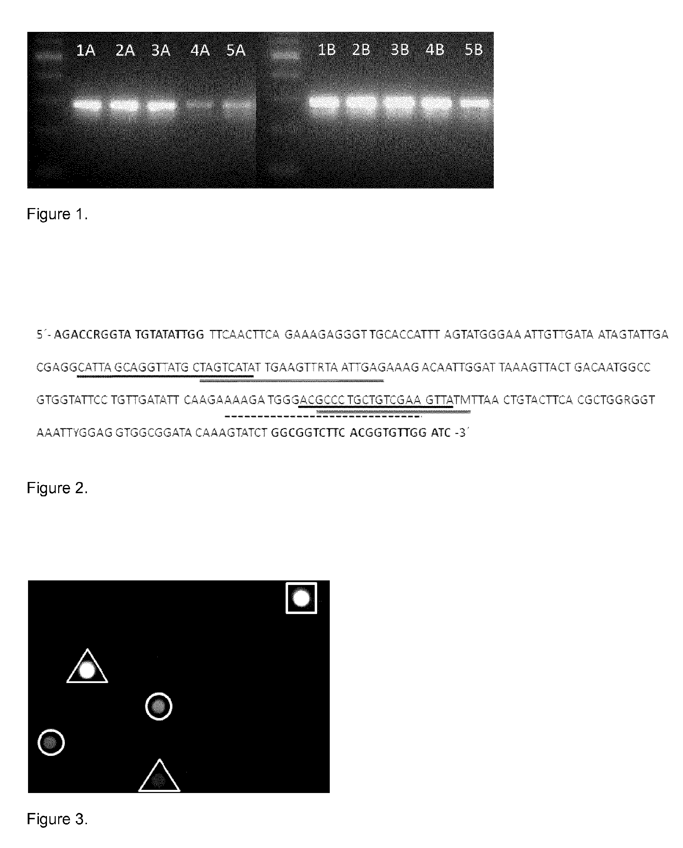

Figure 1 shows the result of the PCR reaction carried out using the primer pair mixture disclosed in WO2004046379 (samples 1A-5A) and the primer pair mixture of the present invention (samples 1 B-5B).

Figure 2 shows an example of a hyper-variable *gyrB* gene region flanked by the conserved sequences of *Staphylococcus epidermidis.* The conserved regions, situated in the 5' and 3' ends of the sequence, are marked in bold. They act as the annealing sites of broad-range *gyrB* and *parE* primers. The hyper-variable region, in which bacterial species or higher-level taxa specific probes have been designed, is between the conserved sequences (five underlined sites represent areas in which the species-specific probes have been designed).

Figure 3 shows the difference of the hybridization efficiency between specific *Pseudomonas aeruginosa gyrB* oligonucleotide probes disclosed in WO2004046379 and the improved probe according to the present invention. The microarray contained three specific *Pseudomonas aeruginosa gyrB* oligonucleotide probes; two disclosed in WO2004046379 (circled spots; SEQ ID NOs: 27/27 and 29/29) and the improved probe according to the present invention (squared spot; SEQ ID NO: 71) that all bound specifically to the target strand. The *Pseudomonas aeruginosa* probes disclosed in WO2004046379 had significantly fainter signal intensities compared to the probe according to the present invention. Two positive control oligonucleotides were also detected (triangle-marked spots).

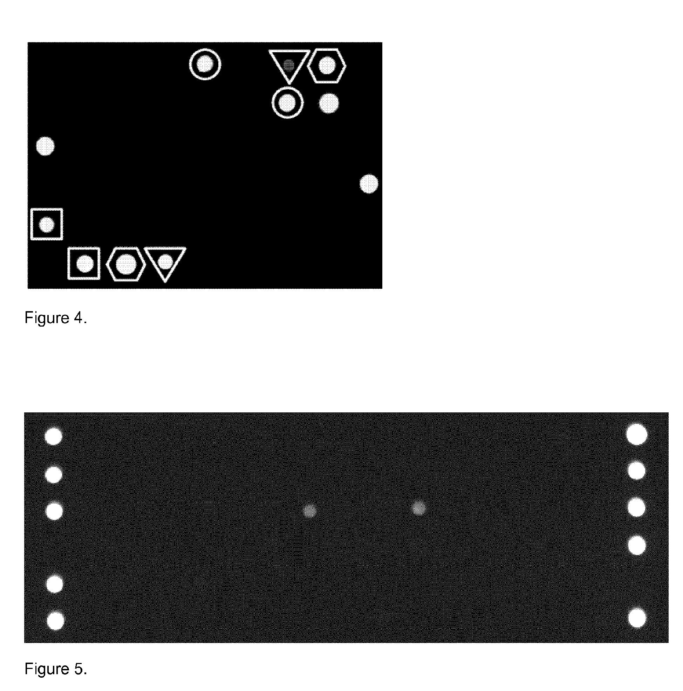

Figure 4 shows the result of microarray hybridization using a sample containing *Klebsiella pneumoniae* and *Enterococcus faecalis* as causative agents. The microarray contained three specific *Klebsiella pneumoniae* probes (circled spots; SEQ ID NOs: 54 and 55, one sequence not disclosed in the present invention), four specific *Enterococcus faecalis* probes (squared spots; SEQ ID NOs: 33 and 116, two sequences not disclosed in the present invention), and two *Enterobacteriaceae* family probes (hexagon-marked spots; SEQ ID NOs: 29 and 30) that bound specifically to the target strands. Two positive control oligonucleotides were also detectable (triangle-marked spots).

Figure 5 shows the result of microarray hybridization using a negative blood culture sample. The microarray contained specific *gyrB* oligonucleotides for the following bacteria *Enterobacter cloacae, Enterococcus faecalis, Enterococcus faecium, Escherichia coli, Klebsiella oxytoca, Klebsiella pneumoniae, Listeria monocytogenes, Neisseria meningitidis, Pseudomonas aeruginosa, Staphylococcus aureus, Staphylococcus epidermidis, Streptococcus agalactiae, Streptococcus pneumoniae, Streptococcus pyogenes.* Furthermore, specific probes for *Enterobacteriaceae family* and *Staphylococcus genus* were on array. No unspecific hybridization was detected. As expected, two positive control oligonucleotides (middle of the array) and ten orientation controls (five on both sides of the array) were detected.

DETAILED DESCRIPTION OF THE INVENTION

The present invention aims to provide improved probes and primers for use in the detection and identification of infection-causing bacteria, especially sepsis-causing bacteria. Examples of bacteria that cause infections, especially sepsis, include both gram-negative bacteria, such as *Acinetobacter baumannii, Bacteroides fragilis* group, *Campylobacter jejuni, Enterobacter aerogenes, Enterobacter cloacae, Escherichia coli, Haemophilus influenzae, Klebsiella oxytoca, Klebsiella pneumoniae, Neisseria meningitidis, Proteus mirabilis, Proteus vulgaris, Pseudomonas aeruginosa, Salmonella enterica* subspecies *enterica,* and *Serratia marcescens,* and gram-positive bacteria, such as *Clostridium perfringens, Enterococcus faecalis, Enterococcus faecium, Listeria monocytogenes, Staphylococcus aureus, Staphylococcus capitis, Staphylococcus epidermidis, Staphylococcus haemolyticus, Staphylococcus hominis, Staphylococcus lugdunensis, Staphylococcus warneri, Staphylococcus xylosus, Stenotrophomonas maltophilia, Streptococcus agalactiae, Streptococcus dysgalactiae, Streptococcus pneumoniae,* and *Streptococcus pyogenes.*

The topoisomerases gyrase B (gyrB or topoisomerase II) and parE proteins (the other subunit of topoisomerase IV) are important molecules for bacteria, because they are needed in the packing of DNA. Certain DNA sequence fragments of these molecules have remained almost unchanged, i.e. conserved, during evolution. In this context, the terms "a conserved region" or "conserved regions" refer to a region or regions of topoisomerase genes or proteins, whose nucleotide sequence or corresponding amino acid sequence has remained nearly unchanged between different bacterial species. Usually these conserved regions are the most important regions for the functioning of the protein. Short DNA sequences can be found in gyrB and parE molecules where the differences between various bacterial species (including closely related bacterial species) are so great that these DNA sequences can be considered as species or higher-level taxa specific. These sequences are hyper-variable sequences, which in this context refer to DNA sequences of the topoisomerase genes which differ in the nucleotide base sequence between different bacteria to an extent affording bacterial species or higher-level taxa specificity and which are situated near the conserved sequences of the topoisomerase genes and optionally limited or marked out by the conserved sequences. The term "hyper-variable sequence" also refers to the amino acid sequence encoded by said hyper-variable DNA sequence.

The present invention provides improved broad-range primers or broad-range primer mixtures that originate from conserved regions of genes encoding topoisomerases, especially the *gyrB* and *parE* genes, and that efficiently amplify DNA of infection-causing bacteria. *GyrB* gene exists as a single copy in all bacteria, while *parE* gene exists mainly in gram-negative bacteria, also as a single copy. Both the *gyrB* and *parE* genes are amplifiable by said primers.

The general design of such primers is disclosed in international patent publication WO2004046379. The primers according to the present invention (Table 1. SEQ ID NOs: 1 and 2) were designed for the same places in the sequence as the primers disclosed in WO2004046379 but the number of degenerated regions was reduced by using inosines and the sequences of clinically relevant sepsis associated bacteria were prioritized in the primer design. The sequences were obtained from the EMBL public sequence database or, in cases where such sequences were not available in public databases, they were produced by cloning the *gyrB* or *parE* gene sequence fragments from the bacterial species or by PCR with primers designed using sequences from closely related bacterial species or partial sequences available in the databases. The number of sequences used was substantially higher than the material used in WO2004046379. The data was systematically analyzed and used in primer design. In the design, inosines were placed into end of the primers since the substitution of universal bases is less destabilizing towards the termini of oligonucleotides than towards the centre. Also, possible formation of inosine-guanine base pairs was avoided.

Furthermore, the primer pair was designed to have partly overlapping melting temperatures. The melting temperature of the broad-range primer depicted in SEQ ID NO: 1 was about 8°C to about 20°C lower than the melting temperature of the broad-range primer depicted in SEQ ID NO: 2. Such a difference in the melting temperatures allows use of a novel DNA amplification method favoring production of single-stranded DNA. The method is performed in two PCR stages having different annealing temperatures. Both the primer mixture depicted in SEQ ID NO: 1 and the primer mixture depicted in SEQ ID NO: 2 anneal and extend a target DNA at the annealing temperature of the first PCR stage, while only the primer mixture depicted in SEQ ID NO: 2 anneals and extends the target DNA at the annealing temperature of the second PCR stage. Thus, the primers according to the present invention possess additional features to their broad-range or universal characteristics allowing effective amplification of single-stranded DNA. In connection with the present invention, production of single-stranded bacterial DNA is preferred although the primers may be used for producing only double-stranded bacterial DNA.

The most successful amplification of pure bacterial DNA was achieved with one of a plurality of different primer pair mixtures tested by various laboratory tests. The *gyrB* and *parE* genes of all tested bacteria could be amplified with the primer mixture according to the present invention (Example 1). The primers thus act as universal or broad-range primers for the bacteria. When compared to the primer pair mixture disclosed in WO2004046379, the primers according to the present invention were clearly more effective and sensitive (Example 2, Figure 1). The bacterial species from culture collections

(ATCC and DSM) along with clinical samples were used in the specificity and sensitivity testing (Table 2).

**Table 1. *gyrB* and *parE* broad-range primers**

| Name of primer | SEQ ID NO: | Sequence 5'->3' |
|---|---|---|
| gB3F | 1 | CGICCIGGKATGTAYATHGG |
| gB4R | 2 | RMICCWACICCRTGYAGICCICC |

In the primer sequences: I represents a base inosine; K represents base G or T; Y represents base C or T; H represents base A or C or T; R represents base A or G; M represents base A or C, and W represents base A or T. The present invention thus concerns primer mixtures, which consist of several different primer alternatives. For example, in the case of primer SEQ ID NO: 1 the mixture includes primers in which K represents G (guanine) or T (thymine).

In addition, oligonucleotide analogues, such as peptide nucleic acids (PNA), or oligonucleotides comprising modified bases can act as primers and thus, are included in the meaning of the primer. The primers may consist of nucleotide sequences depicted in respective SEQ ID NOs or they may comprise additional nucleotides. Furthermore, the primers do not need to be of exactly the same sequence as the template nucleic acid but must be sufficiently complementary to hybridize with the template and retain the capability to amplify the *gyrB* and *parE* genes. Also, various chemical compounds or groups (e.g., amino groups) or other molecules, such as labels, can be attached to the primers, or they can be entirely unmodified. Naturally, reverse and complementary sequences of these oligonucleotide sequences are equally suitable, as is obvious to a person skilled in the art.

Herein the term "complementary sequence" refers to a nucleic acid sequence which consists of complementary nucleotides to the original primer sequence. As known in the art, nucleic acids A and T are complementary to each other, whereas nucleic acids C and G are complementary to each other. In other words, the complementary sequence to T-T-C-A-G (where each letter stands for one of the bases in DNA) is A-A-G-T-C. Thus, the term "complementary sequence" refers to a completely complementary sequence which consists of as many nucleic acids as the original primer sequence.

The term "reverse sequence" refers to a nucleic acid sequence in which the order of nucleotides is reversed as compared to the original primer sequence. For example, reverse sequence to T-T-C-A-G (where each letter stands for one of the bases in DNA) is G-A-C-T-T. Thus, the term "reverse sequence" refers to a completely reversed nucleic acid sequence consisting of as many nucleic acids as the original non-reversed primer sequence.

Thus, each specific SEQ ID NO also refers to reversed or complementary sequences thereof.

Similarly, functional fragments of the previously mentioned oligonucleotide sequences are useful as primers. In this context, the term "functional fragment" refers to truncated nucleotide sequence with or without modifications, capable of hybridizing with and initiating amplification of the target DNA.

The conserved sequences of the first part of the *gyrB* and/or *parE* genes of all studied bacterial species were identified with this mixture of primers. It amplified DNA from clinical samples, and has preserved sufficiently broad specificity, thus enabling the amplification of the *gyrB* and *parE* genes from different bacterial species causing infections, especially sepsis (Example 1). In particular, this mixture of primers can be used to amplify the *gyrB* and *parE* genes of bacteria (Table 2) that are phylogenetically far from each other even in a situation where the sample includes large amounts of foreign (nonbacterial) DNA.

**Table 2. Bacterial strains used in the *gyrB* gene alignment. These bacteria were also used in sensitivity and specificity testing of primers and probes.**

| Bacterial species | Supplier's code (type of sample) |
|---|---|
| *Acinetobacter baumannii* | DSM 30007 |
| *Bacteroides fragilis* | ATCC 25285D |
| *Campylobacter jejuni* | Bacterial culture isolate |
| *Clostridium perfringens* | Bacterial culture isolate |
| *Enterobacter aerogenes* | ATCC 49469 |
| *Enterobacter cloacae* | ATCC 13047 |
| *Enterococcus faecalis* | ATCC 19433 |
| *Enterococcus faecium* | ATCC 19434 |

(continued)

| Bacterial species | Supplier's code (type of sample) |
|---|---|
| *Haemophilus influenzae* | Bacterial culture isolate |
| *Escherichia coli* | DSM 30083 |
| *Klebsiella oxytoca* | DSM 5175 |
| *Klebsiella pneumoniae* | ATCC 700721D |
| *Listeria monocytogenes* | Bacterial culture isolate |
| *Morganella morganii* | Bacterial culture isolate |
| *Neisseria meningitidis* | ATCC 700532D |
| *Proteus mirabilis* | DSM 4479 |
| *Proteus vulgaris* | Bacterial culture isolate |
| *Pseudomonas aeruginosa* | DSM 50071 |
| *Salmonella enterica* subspecies *enterica* | Bacterial culture isolate |
| *Serratia marcescens* | Bacterial culture isolate |
| *Stenotrophomonas maltophilia* | Bacterial culture isolate |
| *Staphylococcus aureus* | ATCC 10832 |
| *Staphylococcus capitis* | ATTC 27840 |
| *Staphylococcus epidermidis* | ATCC 49134 |
| *Staphylococcus haemolyticus* | DSM 20263 |
| *Staphylococcus hominis* | ATCC 25615 |
| *Staphylococcus lugdunensis* | DSM 4805 |
| *Staphylococcus warnerii* | ATCC 17917 |
| *Staphylococcus xylosus* | ATCC 35033 |
| *Streptococcus agalactiae* | ATCC 13813 |
| *Streptococcus anginosus* | DSM 20563 |
| *Streptococcus dysgalactiae* | ATCC BAA-338 |
| *Streptococcus pneumoniae* | DSM 20566 |
| *Streptococcus pyogenes* | DSM 20565 |
| *Yersinia pseudotuberculosis* | Bacterial culture isolate |
| ATCC = American Type Culture Collection<br>DSM = Deutsche Sammlung von Mikroorganismen und Zellkulturen | |

The present invention further provides bacterial species or higher-level taxa specific oligonucleotide probes that originate from hyper-variable regions situated between the conserved regions of genes encoding topoisomerases, especially the *gyrB* and *parE* genes, these hyper-variable regions differing in the base sequences significantly between various bacterial species. With these specific probes, multiple infection-causing bacteria can be simultaneously detected and identified. In connection with the present invention, the term "higher-level taxa" refers to bacterial genera, such as *Staphylococcus* genus and *Proteus* genus, and bacterial families, such as *Enterobacteriaceae* family.

The above-mentioned features were utilized in the design of species or higher-level taxa specific oligonucleotides for multiple bacterial strains as generally disclosed in international patent publication WO2004046379. In the design of such specific probes, a planning strategy based on alignment was used. The *gyrB* or *parE* genes of target bacterial species were aligned with the corresponding genes of the non-targeted bacteria (Example 3). The sequences were obtained from the EMBL public sequence database or, in cases where such sequences were not available in public databases, they were produced by cloning the *gyrB* or *parE* gene sequence fragments from the bacterial species, or they were produced by PCR with primers designed by using sequences from closely related bacterial species or partial sequences available in the databases. An example of the hyper-variable *gyrB* gene region of *Staphylococcus epidermidis,* flanked by the conserved gene regions, is presented in Figure 2.

The specificity of the oligonucleotide probes was tested in laboratory conditions both with pure DNA samples isolated from various bacterial species and with clinical patient samples (Examples 4-5; Table 2). The improved oligonucleotide probes according to the present invention comprise the sequences selected from a group consisting of SEQ ID NOs: 3 to 121 and reversed or complementary sequences or functional fragments thereof. The probes may consist of the nucleotide sequences depicted in respective SEQ ID NOs or they may comprise additional nucleotides. Thus, they can be of different length, and only the desired species or higher-level taxa specificity and functionality of these oligonucleotide probes determine their suitable length in hybridization reactions. The length of the oligonucleotide probe is generally 15 to 60, preferably 15 to 40, and more preferably 20 to 30 nucleotides. Furthermore, the probes can be modified in different ways (e.g., oligonucleotide analogues, such as peptide nucleic acids (PNA), or oligonucleotides comprising modified bases can be used) as long as they retain the species or higher-level taxa specificity. Also, various chemical compounds or groups (e.g., amino groups) or other molecules, such as labels necessary for the detection, can be attached to the probes, or they can be entirely unmodified. The sequences of the preferred bacterial species or higher-level taxa specific probes and their specificities are presented in Table 3, and they have sequences identified with SEQ ID NOs: 3 to 121. Naturally, reversed and complementary sequences of these oligonucleotide sequences are equally suitable, as is obvious to a person skilled in the art.

Herein the term "complementary sequence" refers to a nucleic acid sequence which consists of complementary nucleotides to the original probe sequence. For example, the complementary sequence to C-A-T-G (where each letter stands for one of the bases in DNA) is G-T-A-C. Thus, the term "complementary sequence" refers to a completely complementary sequence which consists of as many nucleic acids as the original probe sequence.

The term "reverse sequence" refers to a nucleic acid sequence in which the order of nucleotides is reversed as compared to the original probe sequence. For example, reverse sequence to T-T-C-A-G (where each letter stands for one of the bases in DNA) is G-A-C-T-T. Thus, the term "reverse sequence" refers to a completely reversed nucleic acid sequence consisting of as many nucleic acids as the original non-reversed probe sequence.

Thus, each specific SEQ ID NO also refers to reversed and complementary sequences thereof.

Similarly, functional fragments of the above mentioned oligonucleotide sequences are useful as probes. In this context, the term "functional fragment" refers to a truncated sequence with or without modifications, capable of hybridizing with targeted bacteria and thus retaining an unchanged species or higher-level taxa specificity.

**Table 3. Species or higher-level taxa specific probes**

| SEQ ID NO | Specificity (gyrB) | Oligo sequence 5'->3' |
|---|---|---|
| 3 | Acinetobacter baumannii | GATGAAGCTTTAGCTGGCCACTG |
| 4 | Acinetobacter baumannii | AGTGTTTCAGATAATGGCC |
| 5 | Acinetobacter baumannii | CAGTGTTTCAGATAATGGCCGC |
| 6 | Acinetobacter baumannii | AATTATTGTCACGATTCACGAGG |
| | | |
| 7 | Bacteroides fragilis group | AGAAATCTGCCCTCGAAGTTGCC |
| 8 | Bacteroides fragilis group | AATCTGCCCTCGAAGTTGCC |
| 9 | Bacteroides fragilis group | GTATTCCGGTAGATTTCCACGAA |
| 10 | Bacteroides fragilis group | GAAGACAACTCTATCACCGTACA |
| 11 | Bacteroides fragilis group | GACCACATCGAAGTGACAATCA |
| 12 | Bacteroides fragilis group | GACCACATCGAAGTGACAATCAA |
| 13 | Bacteroides fragilis group | ACGAAGATAATTCAATCACCGTACA |
| 14 | Bacteroides fragilis group | GAAATCAGCATTGGAAGTAGCAAT |
| | | |
| 15 | Campylobacter jejuni | GATGTAGAAATCACTACTGAAGG |
| 16 | Campylobacter jejuni | TGTATAGTTAGTGATAATGGTCG |
| 17 | Campylobacter jejuni | ATATGCCAACTTTAACTGTTGTTT |

(continued)

| SEQ ID NO | Specificity (gyrB) | Oligo sequence 5'->3' |
|---|---|---|
| 18 | Campylobacter jejuni | TTGATATGCACCCAACTGAAAAT |
|  |  |  |
| 19 | Clostridium perfringens | GTAGTTGATGACGGAAGAGG |
| 20 | Clostridium perfringens | GAAGCATTAGCTGGATACTG |
| 21 | Clostridium perfringens | GGTAGTACTAGTTCAAGAGG |
| 22 | Clostridium perfringens | GGGAAAACCAACTGTAGAAG |
|  |  |  |
| 23 | Enterobacter aerogenes | ACATCGTTGTTACGATCCACAGC |
|  |  |  |
| 24 | Enterobacter aerogenes / Klebsiella pneumoniae | CCGCGGCATCCCAACCGGCATTC |
| 25 | Enterobacter aerogenes / Klebsiella pneumoniae | TCTCCGCGGCGGAAGTTAT |
|  |  |  |
| 26 | Enterobacter cloacae | GATCCATGCGGACAACTCCGT |
| 27 | Enterobacter cloacae | GGTCACGATCCATGCGGACAA |
| 28 | Enterobacter cloacae | AGAGGGCGTATCTGCTGCG |
|  |  |  |
| 29 | Enterobacteriaceae family | GGATGACGGCACCGGTCT |
| 30 | Enterobacteriaceae family | CTATCGACGAAGCGCTCGC |
| 31 | Enterobacteriaceae family | TATCGACGAAGCCCTCGCG |
| 32 | Enterobacteriaceae family | ATCGACGAAGCCCTCGCGGG |
|  |  |  |
| 33 | Enterococcus faecalis | AGCTGTGGAAACAGTGTTTACAG |
|  |  |  |
| 34 | Enterococcus faecium | TATCACCGTTATTGACGACGGTC |
| 35 | Enterococcus faecium | TATCCAGGCAAAAACTGGACGTC |
| 36 | Enterococcus faecium | GGATACCTGTAGATATCCAGGCA |
| 37 | Enterococcus faecium | TCGATCGATGAAGTATTAGCA |
| 38 | Enterococcus faecium | ACTAGTTCTGAGGGTCTGCATCA |
|  |  |  |
| 39 | Escherichia coli | AAATTATCGTCACCATTCACGCC |
| 40 | Escherichia coli | GGCGCGGCATTCCGACCGGTA |
| 41 | Escherichia coli | CGCCGATAACTCTGTCTCTGTA |
| 42 | Escherichia coli | TATCGGCGGCGGAAGTGAT |
| 43 | Escherichia coli | ACGGGCGCGGCATTCCGAC |
| 44 | Escherichia coli | AAGAGGGCGTATCGGCGG |
|  |  |  |
| 45 | Haemophilus influenzae | TCTGTATCGGTGCAAGATGATGG |
| 46 | Haemophilus influenzae | GGCCATTGTTCCGATATTATCG |

(continued)

| SEQ ID NO | Specificity (gyrB) | Oligo sequence 5'->3' |
|---|---|---|
| 47 | Haemophilus influenzae | GTTCCGATATTATCGTGACAA |
| 48 | Haemophilus influenzae | CTGTGGATATTCATCCTGAA |
| 49 | Haemophilus influenzae | ATGCCATTGATGAAGCCCTCGC |
| | | |
| 50 | Enterobacter aerogenes / Klebsiella oxytoca | TTACTGTAAAGACATCGTTGTTAC |
| 51 | Enterobacter aerogenes / Klebsiella oxytoca | ACTGTAAAGACATCGTTGTTACG |
| | | |
| 52 | Klebsiella oxytoca | GCGGTATTCCAACCGGTATTCA |
| 53 | Klebsiella oxytoca | GGCCGCGGTATTCCAACCGGT |
| | | |
| 54 | Klebsiella pneumoniae | TACTGCAAAGATATCGTTGTCA |
| 55 | Klebsiella pneumoniae | CTGCAAAGATATCGTTGTCAC |
| | | |
| 56 | Listeria monocytogenes | TACAATCGAAGCTGATAACAGCA |
| 57 | Listeria monocytogenes | ACTGTTCGTGATAACGGACGTGG |
| 58 | Listeria monocytogenes | GGTCGTCCAACAGTAGAAGT |
| 59 | Listeria monocytogenes | GCAATTGATGAAGCACTTGCT |
| | | |
| 60 | Neisseria meningitidis | AATCACGGTAACGATACACGC |
| 61 | Neisseria meningitidis | CCATTGACGAAGCACTCGCC |
| 62 | Neisseria meningitidis | GTATTGGACAACGCCATTGA |
| 63 | Neisseria meningitidis | GGACATTGCGACAAAATCACG |
| | | |
| 64 | Proteus genus | ATTGTCACTATCCATTCAGATAACTC |
| 65 | Proteus genus | CATGGTGTAGGGGTTTCTGTT |
| | | |
| 66 | Proteus mirabilis | ACGGGTATTCATGAAGAAGAGG |
| 67 | Proteus mirabilis | GTATTCCAACGGGTATTCATGAA |
| 68 | Proteus mirabilis | ATGAAGAAGAGGGCGTTTCTG |
| | | |
| 69 | Proteus vulgaris | ACCAAGAAGAGGGTGTTTCAG |
| 70 | Proteus vulgaris | CCCAGAAGAGGGTGTCTCA |
| | | |
| 71 | Pseudomonas aeruginosa | GATATCCACAAGGAAGAAGGG |
| 72 | Pseudomonas aeruginosa | TCACTGTCCGCGACAATGGAC |
| 73 | Pseudomonas aeruginosa | GCCGGTTAYTGCAGCGAAATCA |
| | | |
| 74 | Salmonella enterica subspecies | TCGTCGTGACTATTCACGCC |

(continued)

| SEQ ID NO | Specificity (gyrB) | Oligo sequence 5'->3' |
|---|---|---|
| | enterica | |
| 75 | Salmonella enterica subspecies | TAACGGATGATGGCCGTGGCATT |
| | enterica | |
| | | |
| 76 | Serratia marcescens | TTCAGCCGCAGAGGTCATCA |
| 77 | Serratia marcescens | ATTCAGGTCACCATCCATGCC |
| 78 | Serratia marcescens | TCAGGTCACCATCCATGCCG |
| 79 | Serratia marcescens | CGACAACTCGGTATCGGTG |
| | | |
| 80 | Staphylococcus aureus | AATAGTATCGATGAAGCATTAGCTG |
| 81 | Staphylococcus aureus | AACGGACGTGGTATCCCAGT |
| 82 | Staphylococcus aureus | GGACGTGGTATCCCAGTTGATAT |
| 83 | Staphylococcus aureus | TCCAGCTGTCGAAGTTATTTTAA |
| | | |
| 84 | Staphylococcus epidermidis | TAGTCATATTGAAGTTRTAATTGAG |
| 85 | Staphylococcus epidermidis | GCCCTGCTGTCGAAGTTATC |
| 86 | Staphylococcus epidermidis | CATTAGCAGGTTATGCTAGTCATA |
| 87 | Staphylococcus epidermidis | ACGCCCTGCTGTCGAAGTTA |
| 88 | Staphylococcus epidermidis | AAAGATGGGACGCCCTGCTGTCGAA |
| | | |
| 89 | Staphylococcus genus | GGCATTCCTGTTGATATTCAAGA |
| 90 | Staphylococcus genus | TCAACTTCAGAAAAAGGTTTACA |
| 91 | Staphylococcus genus | TCAACTTCAGAAAGAGGTTTGCA |
| 92 | Staphylococcus genus | GTTGATAATAGTATTGACGAAGC |
| 93 | Staphylococcus genus | CGCCCAGCAGTTGAAGTTATCT |
| 94 | Staphylococcus genus | CCAGCAGTTGAAGTTATCTTAAC |
| 95 | Staphylococcus genus | CCAGCAGTTGAGGTTATTTTAA |
| 96 | Staphylococcus genus | GGGGCGCCCAGCAGTTGAA |
| | | |
| 97 | Stenotrophomonas maltophilia | TCGGTGTCCGACAACGG |
| 98 | Stenotrophomonas maltophilia | GGCAAGCACGAGCAGATGAG |
| 99 | Stenotrophomonas maltophilia | GCCGAAGTGGTGATGACGGT |
| | | |
| 100 | Streptococcus agalactiae | TTACATTGAACCAGATAACTCTA |
| 101 | Streptococcus agalactiae | TGGAAGACCAGCTGTAGAGACAG |
| 102 | Streptococcus agalactiae | CCTTAGCTGGATTTGCAGGACA |
| 103 | Streptococcus agalactiae | ATTCCGGTAGATATCCAAGAAAA |
| | | |

(continued)

| SEQ ID NO | Specificity (gyrB) | Oligo sequence 5'->3' |
|---|---|---|
| 104 | Streptococcus dysgalactiae | ATCGTCGACAATTCTATTGACGA |
| 105 | Streptococcus dysgalactiae | ATTAGCCGGATTTGCTACTCAC |
| 106 | Streptococcus dysgalactiae | AGGCAGATAATTCAATCACAGT |
| 107 | Streptococcus dysgalactiae | CTACTCACATCCAGGTGTTTAT |
| 108 | Streptococcus dysgalactiae | ATCCAGGTGTTTATCGAGGC |
| | | |
| 109 | Streptococcus pneumoniae | TCCTGCTGTTGAGACCGTCTT |
| 110 | Streptococcus pneumoniae | TCAACCTCAAAAGAAGGTCTTCAC |
| 111 | Streptococcus pneumoniae | AGTTTTTATTGAGCCAGATGAT |
| | | |
| 112 | Streptococcus pyogenes | GTCCCGCCGTTGAAACAGTT |
| 113 | Streptococcus pyogenes | TTTTTACAGTCTTACACGCAGGT |
| 114 | Streptococcus pyogenes | GCAGGTTTTGCCTCTCATATTAAAGTCTT |
| 115 | Streptococcus pyogenes | TTACAGTCTTACACGCAGGTGG |
| | | |
| SEQ ID NO | Specificity (parE) | Oligo sequence 5'->3' |
| 116 | Enterococcus faecalis | CTGGTATCCCAACAGTAGAAGTA |
| 117 | Enterococcus faecium | TTTGGTCGAGGAGGGTACAAAAC |
| 118 | Staphylococcus aureus | GTAAACCGACAGTCGAAGTTATC |
| 119 | Staphylococcus aureus | CGTGGTATGCCAACAGGTAT |
| 120 | Staphylococcus aureus | GTATTTCTATAGAAGATAATGGAC |
| 121 | Staphylococcus epidermidis | TGGTTATGGTGATGCGATTACAGT |
| In the SEQ ID NO: 84, R represents base A or G. | | |

Furthermore, the present invention provides a method of detecting and identifying infection-causing bacteria in a sample to be analyzed. The term "sample" as used herein encompasses a variety of sample types including bacterial and cell cultures, food samples, soil samples, biological fluids, and clinical specimens, such as a tissue sample, a secretion sample, such as sputum, or a brush sample, a blood sample, or another suitable sample, obtained from a patient suspected of suffering from an infectious disease. Especially, the sample to be analyzed is a positive blood culture sample suitable for clinical diagnostic applications.

In one preferred embodiment of the method according to the present invention, bacterial identification is performed using the DNA microarray technology. In this context, a DNA microarray or a DNA chip refers to a small substrate on which known nucleic acid sequences have been attached. If the nucleic acid fragments attached to the microarray are shorter than 100 base pairs (generally about 20 to about 60 base pairs), the microarray is called an oligonucleotide array.

In the method of the present invention, cloning for obtaining bacterial sequences can be performed with any conventionally known method, for example by using commercially available cloning kits (e.g., Qiagen PCR Cloning Kit, or Invitrogen TOPO TA Cloning® Kit). Sequencing of the cloning products can be performed with any sequencer suitable for this purpose (e.g., Applied Biosystems model 373A, 377 or 3100, or Bio-Rad Sequi-Gen® GT), or the products can be sequenced manually. The sequences obtained by cloning, PCR, or bioinformatic means can be analyzed manually or with sequence analysis programs designed for this purpose (e.g., Applied Biosystems Sequencer, or Invitrogen Vector NTI Advance®).

DNA may be isolated from a sample to be analyzed by any conventional method, such as commercial DNA isolation kits (e.g., Roche High Pure PCR Template Preparation Kit, BD Biosciences Clontech NucleoSpin®, or Qiagen QIAamp® DNA Mini kit) or by traditional extraction with phenolchloroform or equivalent organic solvents, either manually or with special devices that are suitable for performing DNA isolation. Automated sample preparation platforms for the isolation of DNA (e.g., bioMérieux NucliSENS® easyMAG™) are preferably used because of their general availability, rapidity, and repeatability. The DNA can also be obtained directly from the sample to be analyzed without separate isolation.

In the method of the present invention, reagents used in the DNA amplification can be any reagents that are conventionally used for the amplification of DNA, and are well known to the persons skilled in the art. Suitable and cost-effective reagents, which are commercially available, include different types of DNA polymerases and buffers therefore (e.g., AmpliTaqGOLD™, AmpliTaq® LD, DyNAzyme™, TaqPlus® Precision, or HotStartTaq®), nucleotides or pre-prepared mixtures of nucleotides (e.g., Sigma, Applied Biosystems, or Amersham Biosystems), and $MgCl_2$ (whereby a product from the manufacturer of the DNA polymerase is generally used).

In the method according to the present invention, a number of known labeling methods can be used in order to produce a labeled target strand, for instance fluorescent labels (e.g., Cy5, Cy3, Cy2, TexasRed, FITC, Alexa 488, TMR, FluorX, ROX, TET, or HEX), radioactive labels (e.g., $^{32}$P, $^{33}$P, or $^{33}$S), chemiluminescent labels (e.g., HiLight Single-Color Kit), and colorimetric detection (e.g., biotin-streptavidin-enzyme conjugate). The invention also comprises the applications in which no label is needed, such as those in which the detection of nucleic acids is based on electric impulses (e.g., Motorola eSensor™).

The equipment used for amplification can be any suitable device (e.g., Biometra® T1 Thermocycler, Applied Biosystems GenAmp® PCR system 2700, or Eppendorf Mastercycler®). Practically all devices and equipment suitable for DNA amplification can be used, and amplification can also be performed manually by transferring reaction tubes from one temperature to another. In addition, amplification can be performed directly on a biochip containing specific wells for the PCR reaction, and specific hybridization area, such as a microarray, whereto the probes and control oligonucleotides can be attached.

Purification of the PCR product can be performed by any commercial method (e.g., Roche High Pure PCR Product Purification Kit, Amersham Biosciences MicroSpin™ S-400 or S-300 HR Columns, or Qiagen QIAquick® PCR-Purification-Kit) or using extraction with an organic solvent. The amplification product can also be used for the hybridization reaction as such without any purification or extraction steps.

In order to form a single-stranded target strand, any suitable method can be used including asymmetric PCR, or exonuclease treatment. The invention also comprises applications in which a double-stranded PCR product can be used in the hybridization reaction e.g. after a denaturation step.

In the hybridization reaction, each single-stranded probe hybridizes with its single-stranded target strand creating a double-stranded structure. In some embodiments the probe and the target strand hybridizes under normal hybridization conditions. A person skilled in the art is able to determine suitable hybridization conditions. The hybridization may be carried out at various hybridization temperatures (generally between 40°C and 70°C), and the time needed to perform the hybridization may vary, depending on the application, from a few minutes to a few days.

When hybridization takes place on a solid support, the probes used in the hybridization can be attached onto the surface of the solid support by covalent or non-covalent binding. Alternatively, other chemical, electrochemical or equivalent attachment methods can be used. A linker or spacer can be attached to the probe. The substrate or support onto which the probes are attached can be manufactured from glass, plastic, polymers, metal, nylon, nitrocellulose, polyacrylamide, silicon, silicon oxide, silica, or a combination thereof, and the size of the substrate can vary from a couple of millimeters to a few centimeters. The surface of the substrate can be coated with aminosilane or other appropriate surface treatment, such as epoxysilane, or alternatively a substrate that does not require any separate surface treatment can be used. Preferred substrates for the oligonucleotide probes are glass and silicon.

The synthesized probes can be printed onto the surface of the microarray support with any commercially available arrayer that is suitable for this purpose (e.g., Qarray-mini arraying system, Lucidea Array Spotter, OmniGrid® arrayer), or they can be pipetted manually onto the surface. Alternatively, the probes can be synthesized directly onto the surface by an appropriate *in situ* synthesis method, such as photolithography or ink-jet technology.

According to the invention, specific probes can be used for identification of infection-causing pathogens, especially bacteria that cause sepsis, in any suitable method, by which hybridization under normal conditions can be demonstrated for instance Southern blot, Northern blot, colony hybridization, fluorescence in situ hybridization (FISH), sequencing, and RT-PCR methods. These methods are well known to the persons skilled in the art and they can be performed both in a solution and on a solid support that binds DNA, such as on a nitrocellulose or nylon membrane or on a glass or silicon based surface.

The hybridization mixture used in hybridization may have a composition different from that presented below in the working Examples, for example, the salt composition and/or the concentration may vary (e.g., 2-4xSSC, or SSPE), or commercially available hybridization solutions can be used (e.g., Sigma ArrayHyb™). In addition, denaturing or stabilizing additives (e.g., formamide, or dimethyl sulfoxide (DMSO)) or substances that decrease nonspecific binding (e.g., bovine serum albumin (BSA), or salmon sperm DNA (ssDNA)) can be used in the hybridization mixture. Hybridization can be carried out at various hybridization temperatures (generally between 40°C and 70°C), and the time needed to perform hybridization can vary, depending on the application, from a few minutes to a few days. Instead of a water bath, hybridization can be carried out for example in an incubator or in a special hybridization device (e.g., GeneTAC HybStation, or Lucidea Slidepro Hybridizer). The posthybridization washing steps can vary in their duration, volume, temperature, and in the composition of the washing solution, and can therefore differ from the exemplified method. The washing of the microarray can also be performed in a separate device. In some cases, washing is not necessary because the microarray can be analyzed immediately after hybridization. In a preferred method, hybridization is performed in a special hybridization device or incubator at 53°C to 58°C for about 10 to 30 minutes.

The microarray can be analyzed by any equipment or reader applicable to this purpose (e.g., GeneTAC UC4, GenePix® Personal 4100A, Agilent DNA Microarray Scanner, or a reader which consists of a light source and a camera). If the target strand is fluorescently labeled, the analysis can also be performed for example by a fluorescent microscope. If a radioactive label has been used, the array or membrane can be analyzed by autoradiography. If hybridization has been carried out on the surface of an electronic microarray and the analysis is thus based on electronic detection, the microarray can be analyzed by special equipment designed for this purpose.

The method of the present invention does not suffer from the problems of the prior art. An amplification step incorporated in the method of the present invention provides high sensitivity. The *gyrB* and *parE* genes from phylogenetically different bacterial species is amplified efficiently with the improved broad-range primers of the invention regardless of the bacterial species being gram-negative or gram-positive (Table 2). Furthermore, the PCR product is short (about 300 base pairs) improving the effectiveness of the amplification reaction.

The improved bacterial species or higher-level taxa specific probes have been designed to hybridize with the *gyrB* or *parE* genes which are considerably more variable than for example the 16S rRNA gene region that has previously been used in bacterial diagnostics. Although bacterial species of normal flora are also amplified efficiently with broad-range primers used in the present method, non-targeted identifications do not occur, because the probes of the invention are bacterial species or higher-level taxa specific and identify those bacteria for which they have been designed. All specific oligonucleotide probes of the present invention were cross-tested with various bacterial species, including many bacterial species that belong to normal flora and closely related clinically relevant bacteria, to optimize the probe design. Thus, the method of the present invention is considerably more sensitive and specific than the previously described methods of similar type.

The present invention further provides a kit for detecting and identifying infection-causing bacteria, especially those causing sepsis. The kit comprises at least one improved DNA primer of the invention as defined above in detail, any suitable and desirable mixture of improved bacterial species or higher-level taxa specific oligonucleotide probe sequences of the invention as defined above in detail, optionally attached to a solid support, optionally at least one suitable positive and/or negative control probe sequence, and optionally reagents required in the amplification, purification, hybridization, washing, and/or detection steps as discussed in detail above. A preferred kit of the invention contains an improved DNA primer mixture comprising sequences selected from a group consisting of SEQ ID NOs: 1 and 2, a chip containing at least one oligonucleotide probe sequence selected from a group consisting of SEQ ID NOs: 3 to 121 attached thereto, optionally at least one positive and/or negative control, and optionally reagents required for performing the method of the invention.

A combination of oligonucleotide probes suitable for use in the present invention comprises at least two oligonucleotide probe sequences selected from a group consisting of SEQ ID NOs: 3 to 121 and reversed or complementary sequences or functional fragments thereof. In one embodiment, the combination may also comprise probes recognizing a single or multiple desired bacteria selected from a group consisting of *Acinetobacter baumannii, Bacteroides fragilis, Campylobacter jejuni, Clostridium perfringens, Enterobacter aerogenes, Enterobacter cloacae, Enterococcus faecalis, Enterococcus faecium, Escherichia coli, Haemophilus influenzae, Klebsiella oxytoca, Klebsiella pneumoniae, Listeria monocytogenes, Neisseria meningitidis, Proteus mirabilis, Proteus vulgaris, Pseudomonas aeruginosa, Salmonella enterica* subspecies *enterica, Serratia marcescens, Staphylococcus aureus, Staphylococcus epidermidis, Streptococcus agalactiae, Streptococcus dysgalactiae, Streptococcus pneumoniae, Streptococcus pyogenes, Stenotrophomonas maltophilia, Proteus* genus, *Staphylococcus* genus, and *Enterobacteriaceae* family.

In the following, the present invention is more precisely illustrated with the Examples. When describing the method, references have been made to different equipment, materials, temperatures, chemicals, or equivalents used in this application. These can naturally be varied in a suitable way in different applications of the invention. Therefore, the present invention and its embodiments are not limited to the Examples described below.

**Example 1. Amplification of DNA by PCR using broad-range primers**

DNA from bacterial culture isolates or clinical patient samples was amplified using the method described below.

DNA was isolated from the analyzed sample using a nucleic acid extraction robot (Nuclisens® easyMAG™, bioMérieux, France). After DNA isolation, the desired target was amplified using PCR. First, a reaction solution was prepared. The PCR reaction mixture contained 1 $\mu$M) of gB3F primer mixture (SEQ ID NO: 1; ordered from Thermo Electron, USA), 1 $\mu$M of Cy5-labeled gB4R primer mixture (SEQ ID NO: 2; ordered from Thermo Electron, USA), 1xHot Start Taq® PCR buffer (Qiagen, Germany), in which $MgCl_2$ was added so that the final concentration was 2.0 mM, 300 $\mu$M of each of dATP, dGTP, dCTP, and dTTP (Finnzymes, Finland), 1.5 g/l BSA (EuroClone, Italy), 0.125 U/$\mu$l Hot Start Taq® DNA polymerase (Qiagen, Germany), and 1.5 $\mu$l isolated DNA in a total volume of 15 $\mu$l.

The PCR was performed using a thermal cycler (Mastercycler®, Eppendorf, Germany). The following PCR program was used: a 15 min denaturation step at 95°C, 36 cycles of 20 sec at 95°C, 35 sec at 52°C, 20 sec at 72°C, after which 10 cycles of 10 sec at 95°C, 30 sec at 67°C, and finally 5 cycles of 10 sec at 95°C and 30 sec at 69°C. After the PCR, the success of amplification of dsDNA and ssDNA was verified by gel electrophoresis using a 2% agarose gel containing SYBR® Green II (Invitrogen, USA).

**Example 2. PCR reaction using two different primer pair mixtures**

The properties of the primers of the present invention were studied with respect to ones disclosed in WO2004046379, which also amplify *gyrB* gene.

A set of five DNA samples extracted from culture isolates of *Staphylococcus epidermidis* was amplified using the reaction mixture described in Example 1. Two parallel reactions using the two different primer mixtures were prepared. In addition of using the primers of the present invention (SEQ ID NOs: 1 to 2), reactions using primers disclosed in WO2004046379 (SEQ ID NOs: 76 to 77) were prepared. The following PCR program was used: a 15 min denaturation step at 95°C, 36 cycles of 10 sec at 96°C, 35 sec at 52°C, and 10 sec at 72°C, after which 5 cycles of 5 sec at 96°C, 30 sec at 65°C, and finally 5 cycles of 5 sec at 96°C and 30 sec at 68°C. The amplification products were analyzed by agarose gel electrophoresis using ethidium bromide.

The results are presented in Figure 1. The primer pair mixture of the present invention produced a strong band from all the samples (1 B-5B). In contrast, amplification performed using the primers disclosed in WO2004046379 produced a strong band from three samples (1A-3A) and a very faint band from two samples (4A-5A). Thus, the primers of the present invention clearly improved the amplification reaction efficiency by providing product from all tested *S. epidermidis* strains.

**Example 3. Design of species or higher-level taxa specific probes**

In the design of bacterial species or higher-level taxa specific oligonucleotides, i.e. probes, a planning strategy based on alignment was used. The *gyrB* and *parE* genes of target bacterial species were aligned with correspondent genes of a few closely related bacterial species. For example, the *gyrB* gene of *Streptococcus pneumoniae* was aligned with *gyrB* genes of *Streptococcus pyogenes, Streptococcus mitis, Streptococcus oralis, Staphylococcus aureus,* and *Fusobacterium necrophorum. S. oralis* and *S. mitis* are closely related to *S. pneumoniae.* Therefore, oligonucleotides designed for S. *pneumoniae* must not react with these normal flora bacteria. Sequences were obtained from the EMBL public sequence database, or they were produced by cloning, or by PCR with primers designed by using sequences from closely related bacterial species or partial sequences available in the databases.

Alignment of the sequences was performed with the BioEdit program using the ClustalW alignment algorithm. The consensus sequence of the alignments was calculated and the suitably conserved regions were identified manually. These regions refer to sequence fragments that are conserved in the genes of the target bacterial species and that are not found at least entirely from the genes of the non-targeted bacteria. Oligonucleotide sequences with the suitable length (15 to 40 nucleotides) were selected from these areas for comparison analyses. The selected oligonucleotide sequences were compared to the EMBL prokaryotic sequence database using the FastA algorithm. The oligonucleotide sequences having at least two mismatches when compared to gyrB or parE sequences of non-targeted bacteria were chosen for further analyses. Theoretical melting temperature ($T_m$) was determined for oligonucleotides and the formation of secondary structures was studied. $T_m$ (°C) was calculated by the equation

$$81.5 + 16.6 \log [Na] + 0.41(\%GC) - 0.61 (\%for)\ 500/N,$$

in which Na is the concentration of monovalent cations (50 mM in calculations), %GC is the proportion of guanine and cytosine, %for is the concentration of formamide (0% in calculations), and N is the length of the oligonucleotide. The formation of secondary structures was studied using a program provided by Sigma-Genosys. The oligonucleotides without strong secondary structures and whose $T_m$ temperature was at least 45°C were chosen for experimental specificity testing.

Oligonucleotide probes were synthesized and simultaneously modified from the 5' terminus ($NH_2$-modified

oligos) (Thermo Electron, USA). The specificity of the probes was tested in the laboratory on microarrays using DNA isolated from different bacterial species (Table 2) and from patient samples. PCR for isolated DNA was performed with primer pair mixture of the present invention (SEQ ID NOs: 1 to 2). Of the tested probes, those which functioned best and had the highest specificity were selected. The sequences and specificity of the bacterial species or higher-level taxa specific probes are presented in Tables 3A and 3B.

**Example 4. Using microarrays with improved primers and probes**

The DNA of the *gyrB* gene isolated from a patient sample containing *Pseudomonas aeruginosa* as a causative agent was processed according to Example 1.

A Cy5-labeled target was hybridized with the silicon-based biochip to which the probes were attached. The hybridization reaction mixture contained around 50 to 100 ng of labeled target, 2xhybridization buffer (consisting of 1 tablet PBS pH 7.4 (Sigma, USA), 0.7 M NaCl (Fluka, Switzerland), 0.1% Tween® 20 (100%, Fluka, Switzerland), 2xDenhardt's (5x, Sigma, USA), 20 $\mu$g/ml salmon sperm DNA (dsDNA) (Amersham, United Kingdom)), hybridization control oligonucleotides, and sterile water. The volume of the reaction mixture was 25 $\mu$l. The 2xhybridization buffer was pre-warmed to 55°C before use. The hybridization reaction mixture was transferred to a hybridization area of the biochip. The hybridization area was sealed with clamps and the biochip was placed in a special hybridization device. During 10 min hybridization at 55°C, a Cy5-labeled ssDNA target bound to the complementary probe sequence immobilized on the microarray creating a labeled double-stranded structure.

After the hybridization step, the biochip was briefly washed in order to remove non-hybridized DNA. The washing step was carried out as follows: in 2xSSC solution for 1 min at 40°C, and in 0.2xSSC solution for 1 min at 40°C. After the washing, the biochip was dried. The biochip was analyzed with an optical reader, which consists of a LED light source and a CCD camera. The detection is based on a fluorescent signal released by the Cy5-labeled gene product and captured by the camera.

An example of the hybridization result is presented in Figure 3. It also illustrates the advantage provided the probes according to the present invention compared to the ones disclosed in WO2004046379. The signal intensities from probes of *Pseudomonas aeruginosa* disclosed in WO2004046379 were weak compared to the strong signal intensities from the improved probe according to the present invention. Thus, the novel design of probes clearly improved the detection of pathogens.

**Example 5. Analysis of patient samples on microarray**

DNA, extracted from a positive blood culture sample of a sepsis patient, was amplified as described in Example 1 using 2 $\mu$M primer concentrations. Both the PCR and the hybridization of the amplified DNA (described in the Example 4) were performed on the same biochip containing wells for the PCR reaction and a specific hybridization area where the probes selected from Table 3 were attached. Two bacterial species, *Klebsiella pneumoniae* and *Enterococcus faecalis,* were detected in the same sample (Figure 4). The identification was in line with the result from the blood culturing.

A negative blood culture sample was also analyzed as described above. Result revealed no signal from any of the bacterial or higher-level taxa specific probes as expected (Figure 5).

Thus, the results demonstrate simultaneous detection of several pathogens, not detectable by probes disclosed in WO2004046379, from the same sample. In addition, no unspecific hybridization from the negative sample was observed.

**Claims**

1. A combination of at least two oligonucleotide probes hybridizing with hyper-variable regions situated between conserved sequences of topoisomerase genes of infection-causing bacteria, said probes comprising a sequence selected from a group consisting of SEQ ID NOs: 3 to 121 and complementary sequences thereof.

2. The combination of oligonucleotides probes according to claim 1, comprising at least one probe comprising a sequence selected from a group consisting of SEQ ID NOs: 39 to 44 and complementary sequences thereof, and at least one probe comprising a sequence selected from a group consisting of SEQ ID NOs: 3 to 38, SEQ ID NOs: 45 to 121, and complementary sequences thereof.

3. An oligonucleotide probe hybridizing with hyper-variable regions situated between conserved sequences of topoisomerase genes of infection-causing bacteria, said probe comprising a sequence selected from a group consisting of SEQ ID NOs: 3 to 121 and complementary sequences thereof.

4. The oligonucleotide probe according to claim 3 wherein the length of the oligonucleotide probe sequence is 15 to 60 nucleotides.

Figure 1.

5'- AGACCRGGTA TGTATATTGG TTCAACTTCA GAAAGAGGGT TGCACCATTT AGTATGGGAA ATTGTTGATA ATAGTATTGA

CGAGGCATTA GCAGGTTATG CTAGTCATAT TGAAGTTRTA ATTGAGAAAG ACAATTGGAT TAAAGTTACT GACAATGGCC

GTGGTATTCC TGTTGATATT CAAGAAAAGA TGGGACGCCC TGCTGTCGAA GTTATMTTAA CTGTACTTCA CGCTGGRGGT

AAATTYGGAG GTGGCGGATA CAAAGTATCT GGCGGTCTTC ACGGTGTTGG ATC -3'

Figure 2.

Figure 3.

Figure 4.

Figure 5.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 613502 B1
- US 6001564 A
- US 20020055101 A

- WO 2004046379 A

### Non-patent literature cited in the description

- **DAUGA.** *Int. J. Syst. Evol. Microbiol.,* 2002, vol. 52, 531-547

SEQUENCE LISTING

<110> Mobidiag Oy

<120> Nucleic acid probes and broad-range primers

<130> 2062104EP/DIV

<160> 121

<170> PatentIn version 3.2

<210> 1
<211> 20
<212> DNA
<213> Artificial

<220>
<223> Primer

<220>
<221> misc_feature
<222> (3)..(3)
<223> n represents inosine

<220>
<221> misc_feature
<222> (6)..(6)
<223> n represents inosine

<220>
<221> misc_feature
<222> (9)..(9)
<223> n represents G or T

<220>
<221> misc_feature
<222> (15)..(15)
<223> n represents C or T

<220>
<221> misc_feature
<222> (18)..(18)
<223> n represents A, C or T

<400> 1
cgnccnggna tgtanatngg                                                    20

<210> 2
<211> 23
<212> DNA
<213> Artificial

<220>
<223> Primer

<220>
<221> misc_feature
<222> (1)..(1)
<223> n represents A or G

```
<220>
<221>  misc_feature
<222>  (2)..(2)
<223>  n represents A or C

<220>
<221>  misc_feature
<222>  (3)..(3)
<223>  n represents inosine

<220>
<221>  misc_feature
<222>  (6)..(6)
<223>  n represents A or T

<220>
<221>  misc_feature
<222>  (9)..(9)
<223>  n represents inosine

<220>
<221>  misc_feature
<222>  (12)..(12)
<223>  n represents A or G

<220>
<221>  misc_feature
<222>  (15)..(15)
<223>  n represents C or T

<220>
<221>  misc_feature
<222>  (18)..(18)
<223>  n represents inosine

<220>
<221>  misc_feature
<222>  (21)..(21)
<223>  n represents inosine

<400>  2
nnnccnacnc cntgnagncc ncc                                           23


<210>  3
<211>  23
<212>  DNA
<213>  Acinetobacter baumannii

<400>  3
gatgaagctt tagctggcca ctg                                           23


<210>  4
<211>  19
<212>  DNA
<213>  Acinetobacter baumannii

<400>  4
agtgtttcag ataatggcc                                                19
```

<210> 5
<211> 22
<212> DNA
<213> Acinetobacter baumannii

<400> 5
cagtgtttca gataatggcc gc                                                          22


<210> 6
<211> 23
<212> DNA
<213> Acinetobacter baumannii

<400> 6
aattattgtc acgattcacg agg                                                         23


<210> 7
<211> 23
<212> DNA
<213> Bacteroides fragilis group

<400> 7
agaaatctgc cctcgaagtt gcc                                                         23


<210> 8
<211> 20
<212> DNA
<213> Bacteroides fragilis group

<400> 8
aatctgccct cgaagttgcc                                                             20


<210> 9
<211> 23
<212> DNA
<213> Bacteroides fragilis group

<400> 9
gtattccggt agatttccac gaa                                                         23


<210> 10
<211> 23
<212> DNA
<213> Bacteroides fragilis group

<400> 10
gaagacaact ctatcaccgt aca                                                         23


<210> 11
<211> 22
<212> DNA
<213> Bacteroides fragilis group

<400> 11
gaccacatcg aagtgacaat ca                                                          22

<210> 12
<211> 23
<212> DNA
<213> Bacteroides fragilis group

<400> 12
gaccacatcg aagtgacaat caa                                                    23

<210> 13
<211> 25
<212> DNA
<213> Bacteroides fragilis group

<400> 13
acgaagataa ttcaatcacc gtaca                                                  25

<210> 14
<211> 24
<212> DNA
<213> Bacteroides fragilis group

<400> 14
gaaatcagca ttggaagtag caat                                                   24

<210> 15
<211> 23
<212> DNA
<213> Campylobacter jejuni

<400> 15
gatgtagaaa tcactactga agg                                                    23

<210> 16
<211> 23
<212> DNA
<213> Campylobacter jejuni

<400> 16
tgtatagtta gtgataatgg tcg                                                    23

<210> 17
<211> 24
<212> DNA
<213> Campylobacter jejuni

<400> 17
atatgccaac tttaactgtt gttt                                                   24

<210> 18
<211> 23
<212> DNA
<213> Campylobacter jejuni

<400> 18
ttgatatgca cccaactgaa aat                                                    23

```
<210>  19
<211>  20
<212>  DNA
<213>  Clostridium perfringens

<400>  19
gtagttgatg acggaagagg                                                    20


<210>  20
<211>  20
<212>  DNA
<213>  Clostridium perfringens

<400>  20
gaagcattag ctggatactg                                                    20


<210>  21
<211>  20
<212>  DNA
<213>  Clostridium perfringens

<400>  21
ggtagtacta gttcaagagg                                                    20


<210>  22
<211>  20
<212>  DNA
<213>  Clostridium perfringens

<400>  22
gggaaaacca actgtagaag                                                    20


<210>  23
<211>  23
<212>  DNA
<213>  Enterobacter aerogenes

<400>  23
acatcgttgt tacgatccac agc                                                23


<210>  24
<211>  23
<212>  DNA
<213>  Enterobacter aerogenes / Klebsiella pneumoniae

<400>  24
ccgcggcatc ccaaccggca ttc                                                23


<210>  25
<211>  19
<212>  DNA
<213>  Enterobacter aerogenes / Klebsiella pneumoniae

<400>  25
tctccgcggc ggaagttat                                                     19
```

<210> 26
<211> 21
<212> DNA
<213> Enterobacter cloacae

<400> 26
gatccatgcg gacaactccg t                                              21

<210> 27
<211> 21
<212> DNA
<213> Enterobacter cloacae

<400> 27
ggtcacgatc catgcggaca a                                              21

<210> 28
<211> 19
<212> DNA
<213> Enterobacter cloacae

<400> 28
agagggcgta tctgctgcg                                                 19

<210> 29
<211> 18
<212> DNA
<213> Enterobacteriaceae family

<400> 29
ggatgacggc accggtct                                                  18

<210> 30
<211> 19
<212> DNA
<213> Enterobacteriaceae family

<400> 30
ctatcgacga agcgctcgc                                                 19

<210> 31
<211> 19
<212> DNA
<213> Enterobacteriaceae family

<400> 31
tatcgacgaa gccctcgcg                                                 19

<210> 32
<211> 20
<212> DNA
<213> Enterobacteriaceae family

<400> 32
atcgacgaag ccctcgcggg                                                20

<210> 33
<211> 23
<212> DNA
<213> Enterococcus faecalis

<400> 33
agctgtggaa acagtgttta cag                                                23


<210> 34
<211> 23
<212> DNA
<213> Enterococcus faecium

<400> 34
tatcaccgtt attgacgacg gtc                                                23


<210> 35
<211> 23
<212> DNA
<213> Enterococcus faecium

<400> 35
tatccaggca aaaactggac gtc                                                23


<210> 36
<211> 23
<212> DNA
<213> Enterococcus faecium

<400> 36
ggatacctgt agatatccag gca                                                23


<210> 37
<211> 21
<212> DNA
<213> Enterococcus faecium

<400> 37
tcgatcgatg aagtattagc a                                                  21


<210> 38
<211> 23
<212> DNA
<213> Enterococcus faecium

<400> 38
actagttctg agggtctgca tca                                                23


<210> 39
<211> 23
<212> DNA
<213> Escherichia coli

<400> 39
aaattatcgt caccattcac gcc                                                23

<210> 40
<211> 21
<212> DNA
<213> Escherichia coli

<400> 40
ggcgcggcat tccgaccggt a                                              21


<210> 41
<211> 22
<212> DNA
<213> Escherichia coli

<400> 41
cgccgataac tctgtctctg ta                                             22


<210> 42
<211> 19
<212> DNA
<213> Escherichia coli

<400> 42
tatcggcggc ggaagtgat                                                 19


<210> 43
<211> 19
<212> DNA
<213> Escherichia coli

<400> 43
acgggcgcgg cattccgac                                                 19


<210> 44
<211> 18
<212> DNA
<213> Escherichia coli

<400> 44
aagagggcgt atcggcgg                                                  18


<210> 45
<211> 23
<212> DNA
<213> Haemophilus influenzae

<400> 45
tctgtatcgg tgcaagatga tgg                                            23


<210> 46
<211> 22
<212> DNA
<213> Haemophilus influenzae

<400> 46
ggccattgtt ccgatattat cg                                             22

<210> 47
<211> 21
<212> DNA
<213> Haemophilus influenzae

<400> 47
gttccgatat tatcgtgaca a                                                    21


<210> 48
<211> 20
<212> DNA
<213> Haemophilus influenzae

<400> 48
ctgtggatat tcatcctgaa                                                      20


<210> 49
<211> 22
<212> DNA
<213> Haemophilus influenzae

<400> 49
atgccattga tgaagccctc gc                                                   22


<210> 50
<211> 24
<212> DNA
<213> Enterobacter aerogenes / Klebsiella oxytoca

<400> 50
ttactgtaaa gacatcgttg ttac                                                 24


<210> 51
<211> 23
<212> DNA
<213> Enterobacter aerogenes / Klebsiella oxytoca

<400> 51
actgtaaaga catcgttgtt acg                                                  23


<210> 52
<211> 22
<212> DNA
<213> Klebsiella oxytoca

<400> 52
gcggtattcc aaccggtatt ca                                                   22


<210> 53
<211> 21
<212> DNA
<213> Klebsiella oxytoca

<400> 53
ggccgcggta ttccaaccgg t                                                    21

```
<210>   54
<211>   22
<212>   DNA
<213>   Klebsiella pneumoniae

<400>   54
tactgcaaag atatcgttgt ca                                                        22


<210>   55
<211>   21
<212>   DNA
<213>   Klebsiella pneumoniae

<400>   55
ctgcaaagat atcgttgtca c                                                         21


<210>   56
<211>   23
<212>   DNA
<213>   Listeria monocytogenes

<400>   56
tacaatcgaa gctgataaca gca                                                       23


<210>   57
<211>   23
<212>   DNA
<213>   Listeria monocytogenes

<400>   57
actgttcgtg ataacggacg tgg                                                       23


<210>   58
<211>   20
<212>   DNA
<213>   Listeria monocytogenes

<400>   58
ggtcgtccaa cagtagaagt                                                           20


<210>   59
<211>   21
<212>   DNA
<213>   Listeria monocytogenes

<400>   59
gcaattgatg aagcacttgc t                                                         21


<210>   60
<211>   21
<212>   DNA
<213>   Neisseria meningitidis

<400>   60
aatcacggta acgatacacg c                                                         21
```

```
<210>    61
<211>    20
<212>    DNA
<213>    Neisseria meningitidis

<400>    61
ccattgacga agcactcgcc                                              20


<210>    62
<211>    20
<212>    DNA
<213>    Neisseria meningitidis

<400>    62
gtattggaca acgccattga                                             20


<210>    63
<211>    21
<212>    DNA
<213>    Neisseria meningitidis

<400>    63
ggacattgcg acaaaatcac g                                           21


<210>    64
<211>    26
<212>    DNA
<213>    Proteus genus

<400>    64
attgtcacta tccattcaga taactc                                     26


<210>    65
<211>    21
<212>    DNA
<213>    Proteus genus

<400>    65
catggtgtag gggtttctgt t                                          21


<210>    66
<211>    22
<212>    DNA
<213>    Proteus mirabilis

<400>    66
acgggtattc atgaagaaga gg                                         22


<210>    67
<211>    23
<212>    DNA
<213>    Proteus mirabilis

<400>    67
gtattccaac gggtattcat gaa                                        23
```

27

<210> 68
<211> 21
<212> DNA
<213> Proteus mirabilis

<400> 68
atgaagaaga gggcgtttct g                                                        21


<210> 69
<211> 21
<212> DNA
<213> Proteus vulgaris

<400> 69
accaagaaga gggtgtttca g                                                        21


<210> 70
<211> 21
<212> DNA
<213> Proteus vulgaris

<400> 70
accaagaaga gggtgtttca g                                                        21


<210> 71
<211> 21
<212> DNA
<213> Pseudomonas aeruginosa

<400> 71
gatatccaca aggaagaagg g                                                        21


<210> 72
<211> 21
<212> DNA
<213> Pseudomonas aeruginosa

<400> 72
tcactgtccg cgacaatgga c                                                        21


<210> 73
<211> 22
<212> DNA
<213> Pseudomonas aeruginosa

<400> 73
gccggttayt gcagcgaaat ca                                                       22


<210> 74
<211> 20
<212> DNA
<213> Salmonella enterica subspecies enterica

<400> 74
tcgtcgtgac tattcacgcc                                                          20

<210> 75
<211> 23
<212> DNA
<213> Salmonella enterica subspecies enterica

<400> 75
taacggatga tggccgtggc att                                          23


<210> 76
<211> 20
<212> DNA
<213> Serratia marcescens

<400> 76
ttcagccgca gaggtcatca                                              20


<210> 77
<211> 21
<212> DNA
<213> Serratia marcescens

<400> 77
attcaggtca ccatccatgc c                                            21


<210> 78
<211> 20
<212> DNA
<213> Serratia marcescens

<400> 78
tcaggtcacc atccatgccg                                              20


<210> 79
<211> 19
<212> DNA
<213> Serratia marcescens

<400> 79
cgacaactcg gtatcggtg                                               19


<210> 80
<211> 25
<212> DNA
<213> Staphylococcus aureus

<400> 80
aatagtatcg atgaagcatt agctg                                        25


<210> 81
<211> 20
<212> DNA
<213> Staphylococcus aureus

<400> 81
aacggacgtg gtatcccagt                                              20

```
<210>    82
<211>    23
<212>    DNA
<213>    Staphylococcus aureus

<400>    82
ggacgtggta tcccagttga tat                                             23


<210>    83
<211>    23
<212>    DNA
<213>    Staphylococcus aureus

<400>    83
tccagctgtc gaagttattt taa                                            23


<210>    84
<211>    25
<212>    DNA
<213>    Staphylococcus epidermidis


<220>
<221>    misc_feature
<222>    (17)..(17)
<223>    n represents A or G

<400>    84
tagtcatatt gaagttntaa ttgag                                          25


<210>    85
<211>    20
<212>    DNA
<213>    Staphylococcus epidermidis

<400>    85
gccctgctgt cgaagttatc                                                20


<210>    86
<211>    24
<212>    DNA
<213>    Staphylococcus epidermidis

<400>    86
cattagcagg ttatgctagt cata                                           24


<210>    87
<211>    20
<212>    DNA
<213>    Staphylococcus epidermidis

<400>    87
acgccctgct gtcgaagtta                                                20


<210>    88
<211>    25
<212>    DNA
```

```
<213>   Staphylococcus epidermidis

<400>   88
aaagatggga cgccctgctg tcgaa                                              25


<210>   89
<211>   23
<212>   DNA
<213>   Staphylococcus genus

<400>   89
ggcattcctg ttgatattca aga                                               23


<210>   90
<211>   23
<212>   DNA
<213>   Staphylococcus genus

<400>   90
tcaacttcag aaaaaggttt aca                                               23


<210>   91
<211>   23
<212>   DNA
<213>   Staphylococcus genus

<400>   91
tcaacttcag aaagaggttt gca                                               23


<210>   92
<211>   23
<212>   DNA
<213>   Staphylococcus genus

<400>   92
gttgataata gtattgacga agc                                               23


<210>   93
<211>   22
<212>   DNA
<213>   Staphylococcus genus

<400>   93
cgcccagcag ttgaagttat ct                                                22


<210>   94
<211>   23
<212>   DNA
<213>   Staphylococcus genus

<400>   94
ccagcagttg aagttatctt aac                                               23


<210>   95
<211>   22
<212>   DNA
```

<213> Staphylococcus genus

<400> 95
ccagcagttg aggttatttt aa                                                         22


<210> 96
<211> 19
<212> DNA
<213> Staphylococcus genus

<400> 96
ggggcgccca gcagttgaa                                                             19


<210> 97
<211> 17
<212> DNA
<213> Stenotrophomonas maltophilia

<400> 97
tcggtgtccg acaacgg                                                               17


<210> 98
<211> 20
<212> DNA
<213> Stenotrophomonas maltophilia

<400> 98
ggcaagcacg agcagatgag                                                            20


<210> 99
<211> 20
<212> DNA
<213> Stenotrophomonas maltophilia

<400> 99
gccgaagtgg tgatgacggt                                                            20


<210> 100
<211> 23
<212> DNA
<213> Streptococcus agalactiae

<400> 100
ttacattgaa ccagataact cta                                                        23


<210> 101
<211> 23
<212> DNA
<213> Streptococcus agalactiae

<400> 101
tggaagacca gctgtagaga cag                                                        23


<210> 102
<211> 22
<212> DNA

<213>    Streptococcus agalactiae

<400>    102
ccttagctgg atttgcagga ca                                                22


<210>    103
<211>    23
<212>    DNA
<213>    Streptococcus agalactiae

<400>    103
attccggtag atatccaaga aaa                                               23


<210>    104
<211>    23
<212>    DNA
<213>    Streptococcus dysgalactiae

<400>    104
atcgtcgaca attctattga cga                                               23


<210>    105
<211>    22
<212>    DNA
<213>    Streptococcus dysgalactiae

<400>    105
attagccgga tttgctactc ac                                                22


<210>    106
<211>    22
<212>    DNA
<213>    Streptococcus dysgalactiae

<400>    106
aggcagataa ttcaatcaca gt                                                22


<210>    107
<211>    22
<212>    DNA
<213>    Streptococcus dysgalactiae

<400>    107
ctactcacat ccaggtgttt at                                                22


<210>    108
<211>    20
<212>    DNA
<213>    Streptococcus dysgalactiae

<400>    108
atccaggtgt ttatcgaggc                                                   20


<210>    109
<211>    21
<212>    DNA

<213> Streptococcus pneumoniae

<400> 109
tcctgctgtt gagaccgtct t                                        21

<210> 110
<211> 24
<212> DNA
<213> Streptococcus pneumoniae

<400> 110
tcaacctcaa aagaaggtct tcac                                     24

<210> 111
<211> 22
<212> DNA
<213> Streptococcus pneumoniae

<400> 111
agtttttatt gagccagatg at                                       22

<210> 112
<211> 20
<212> DNA
<213> Streptococcus pyogenes

<400> 112
gtcccgccgt tgaaacagtt                                          20

<210> 113
<211> 23
<212> DNA
<213> Streptococcus pyogenes

<400> 113
tttttacagt cttacacgca ggt                                      23

<210> 114
<211> 29
<212> DNA
<213> Streptococcus pyogenes

<400> 114
gcaggttttg cctctcatat taaagtctt                                29

<210> 115
<211> 22
<212> DNA
<213> Streptococcus pyogenes

<400> 115
ttacagtctt acacgcaggt gg                                       22

<210> 116
<211> 23
<212> DNA

```
<213>  Enterococcus faecalis

<400>  116
ctggtatccc aacagtagaa gta                                        23


<210>  117
<211>  23
<212>  DNA
<213>  Enterococcus faecium

<400>  117
tttggtcgag gagggtacaa aac                                        23


<210>  118
<211>  23
<212>  DNA
<213>  Staphylococcus aureus

<400>  118
gtaaaccgac agtcgaagtt atc                                        23


<210>  119
<211>  20
<212>  DNA
<213>  Staphylococcus aureus

<400>  119
cgtggtatgc caacaggtat                                            20


<210>  120
<211>  24
<212>  DNA
<213>  Staphylococcus aureus

<400>  120
gtatttctat agaagataat ggac                                       24


<210>  121
<211>  24
<212>  DNA
<213>  Staphylococcus epidermidis

<400>  121
tggttatggt gatgcgatta cagt                                       24
```